# EUROPEAN PATENT SPECIFICATION

(11) **EP 4 222 823 B1**
(45) Date of publication and mention of the grant of the patent: **19.11.2025**
(21) Application number: 21783426.6
(22) Date of filing: 22.09.2021
(51) Int. Cl.: H01R 13/52, H01R 13/64

(54) **MULTI-USE MEDICAL DEVICES COMPRISING SHAPE-MEMORY ALLOY TO BLOCK USE BEFORE DISINFECTION/STERILIZATION**
MEDIZINISCHE MEHRZWECKVORRICHTUNGEN MIT FORMGEDÄCHTNISLEGIERUNG ZUR BLOCKIERUNG DER VERWENDUNG VOR DER DESINFEKTION/STERILISATION
DISPOSITIFS MÉDICAUX POLYVALENTS COMPRENANT UN ALLIAGE À MÉMOIRE DE FORME PERMETTANT DE BLOQUER L'UTILISATION AVANT DÉSINFECTION/STÉRILISATION

(30) Priority: 01.10.2020 US 202063086124 P
(43) Date of publication of application: 09.08.2023
(73) Proprietor: Koninklijke Philips N.V., 5656 AG Eindhoven (NL)
(72) Inventor: JOYE, Neil, Francis, 5656 AE Eindhoven (NL); MUEHLSTEFF, Jens, 5656 AE Eindhoven (NL); VAN ROOIJ, Johannes, Antonius, 5656 AE Eindhoven (NL); PELSSERS, Eduard, Gerard, Marie, 5656 AE Eindhoven (NL)
(74) Representative: Philips Intellectual Property & Standards
(86) International application number: PCT/EP2021/076010
(87) International publication number: WO 2022/069306

(56) References cited:
- WO-A1-2021/115859
- JP-A- H1 094 599
- US-A- 4 449 518
- US-A- 4 950 173
- US-A1- 2007 208 451
- US-A1- 2018 154 135
- US-B1- 6 295 330

## Description

### BACKGROUND

Medical consumables such as electrocardiograph (ECG) lead sets, SpO₂ sensors, and blood pressure cuffs are typically available in two options: multi-patient use (i.e., reusable) and single-patient use (i.e., disposable). As the name describes, a multi-patient use consumable is used on many different patients over the lifetime of the consumable. These consumables are cleaned and/or disinfected between each use.

Typically, disinfectant wipes are used by the hospital staff for cleaning these consumables. However, this approach has certain drawbacks. For example, cleaning the consumables is time consuming and adds further duties to medical personnel daily obligations. Moreover, many consumables are difficult to clean. For example, the existence of small ridges and clamps in some ECG lead wires causes difficulty in cleaning and disinfecting the ECG lead wires thoroughly. It has for example been shown that bacteria are common on reusables, cleaned lead wires. These consumables are therefore seen as a potential source of Hospital Acquired Infections (HAI).

As an alternative, hospitals can opt for single-patient use consumables. With this type of consumables, patients always receive clean devices, which limit the risk of HAI. They are also seen as convenient since such consumables are always ready to be used and no hand cleaning is required after each use. However, the overall cost of the use of consumable devices, when compared to multi-patient use consumables is larger. Moreover, single-patient consumables can result in unacceptable increases in electronic and biohazard waste. In addition, health care facilities relying on single-patient use consumables will need to regularly restock supplies of these components, exposing the health care facilities to shortages in supply of the components, and delays in delivery by suppliers, to name only a few drawbacks.

What is needed, therefore, are medical components that overcome at least the shortcomings of known components discussed above.

US 4 449 518 A discloses a disinfection indicator mounted on medical equipment which is inserted onto a channel for forceps and repeatedly used after thermal sterilization. The disinfection indicator includes a deformable portion made of a shape memory alloy.

US 2018/0154135 A1 discloses systems and methods for treating a gastrointestinal condition of a patient which includes minimally invasively implanting a modular stimulator system in a patient's treatment site.

### SUMMARY

According to an aspect of the present disclosure, a method of ensuring a medical connector for a medical device is disinfected, or sterilized, or both is disclosed. The method accoding to claim 1 comprises: receiving the medical connector in a blocked state, wherein a component of the medical connector comprises a shape-memory alloy (SMA); and heating the medical connector to a sufficient temperature to change a shape of the SMA to an unblocked state.

According to another aspect of the present disclosure, a medical device is disclosed according to claim 9. The medical device comprises: a medical connector comprising a shape memory alloy (SMA) adapted to engage a mating connector in an unblocked state, wherein upon disengaging the medical connector the SMA is in a blocked state.

### BRIEF DESCRIPTION OF THE DRAWINGS

The representative embodiments are better understood from the following detailed description when read with the accompanying drawing figures. It is emphasized that the various features are not necessarily drawn to scale. In fact, the dimensions may be arbitrarily increased or decreased for clarity of discussion. Wherever applicable and practical, like reference numerals refer to like elements.
Fig. 1 is a flow-chart of a method to prevent a medical device from being connected to an apparatus before being unblocked by disinfection or sterilization, or both, in accordance with a representative embodiment.
Figs. 2A-2C are simplified cross-sectional views of a medical connector comprising shape-memory alloy (SMA) showing states of SMA in three stages (A, B, C) of use according to a representative embodiment.
Fig. 3 is a simplified end-on view of the connector of the medical device of Fig. 2A in an unblocked state, in accordance with a representative embodiment.
Fig. 4A is a perspective view of an electrocardiograph (ECG) lead set comprising electrically shielding electrodes in accordance with a representative embodiment.
Figs. 4B-4D are simplified cross-sectional views of the ECG lead set depicted in Fig. 4 showing states of SMA in three stages of use of the ECG lead set in accordance with a representative embodiment.
Figs. 5A-5H are simplified cross-sectional views of an electrical connector for a medical device showing states of SMA in various stages of use of the electrical connector in accordance with a representative embodiment.

### DETAILED DESCRIPTION

Descriptions of known systems, devices, materials, methods of operation and methods of manufacture may be omitted so as to avoid obscuring the description of the representative embodiments. Nonetheless, systems, devices, materials and methods that are within the purview of one of ordinary skill in the art are within the scope of the present teachings and may be used in accordance with the representative embodiments.

The present disclosure, through one or more of its various aspects, representative embodiments and/or specific features or sub-components, is intended to bring out one or more of the advantages as specifically noted below. For purposes of explanation representative embodiments disclosing specific details are set forth in order to provide a thorough understanding of an embodiment according to the present teachings. Moreover, descriptions of well-known apparatuses and methods may be omitted so as to not obscure the description of the example embodiments. Such methods and apparatuses are within the scope of the present disclosure.

Generally, and as described below in connection with various representative embodiments, the present teachings are directed to including a shape-memory alloy (SMA) element, or a bi-metal element in the medical connector of the consumable (sometimes referred to herein as a medical connector). These consumables may be, for example, but are certainly not limited to, electrocardiograph (ECG) lead sets, SpO₂ sensors, temperature probes, as well as other devices that must be sterilized, or disinfected, or both, before being reconnected to a mating connector of a medical component/instrument. Notably, and as described more fully below, disinfection and sterilization are carried out at different temperatures. The most common sterilization method is steam sterilization (e.g., using an autoclave) that occurs at higher temperatures than in an automated washer. The SMA transformation temperature is, therefore, not set above the sterilization temperature. Notably, in certain representative embodiments only the disinfection step is required/mandatory and the sterilization step optional.

When detached from a mating connector (e.g., a connector of a patient monitor), the SMA element is deformed to a blocked state. The deformed shape of the SMA element in the blocked state prevents the medical connector from being reconnected to its mating connector (not shown). In its blocked state, the medical connector is heated to at least its transformation temperature, and the SMA element is reformed to its original shape so it can again be reconnected to its mating connector. In this state, the medical connector is unblocked. By the present teachings, the transformation temperature of the SMA is chosen such that when the consumable is subjected to the high temperatures of disinfecting and/or sterilization methods, such as automated cleaning or steam sterilization, it returns to its pre-deformed shape. In that situation, the medical connector can be reinserted in its corresponding mating connector (e.g. reconnected to a patient monitor).

As described in connection with various representative embodiments below, the present teachings include providing consumables (e.g. ECG lead sets, SpO₂ sensors, temperature probes, etc.) that can be cleaned, disinfected, and sterilized at clinical locations (e.g., hospitals or medical clinics), or alternatively at a third-party location. For example, since many hospitals and clinics have a Central Sterilization Facility (CSF) where medical devices are disinfected in an automated washer and sterilized in an autoclave. Such automated washer and sterilization steps may be defined by the following standards: ISO 15883 for the automated cleaning; and ISO 17665 for the autoclave step. In accordance with various representative embodiments, the transformation temperature of the SMA element of the medical connector is selected to be greater than the maximum expected ambient room temperature (typically in the range of 25°C - 30°C), and no greater than the temperature required by ISO 15883, if only automated cleaning and disinfection is required; or no greater than the temperature required by ISO 17665 if steam sterilization in an autoclave is required or mandatory. As such, the temperature of the automated washer and/or autoclave needs to be larger than the SMA transformation temperature.

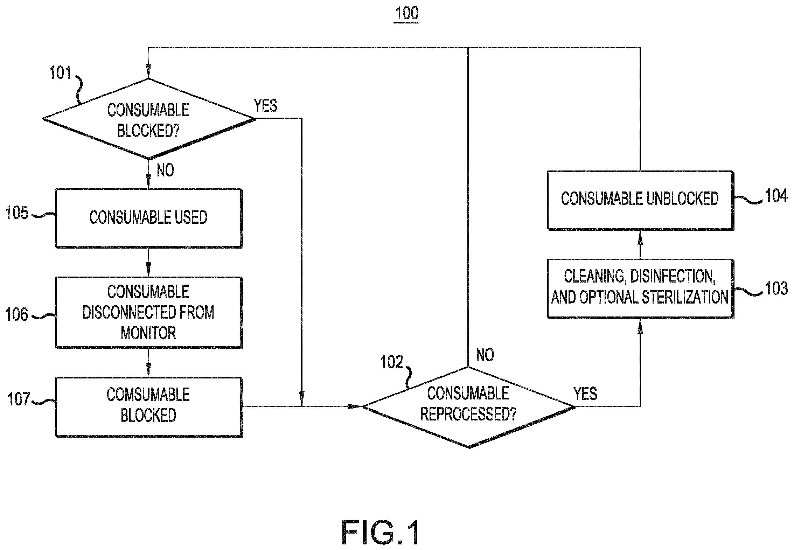

Fig. 1 is a flow-chart of a method 100 to prevent a medical connector from being connected to an apparatus before being unblocked by disinfection or sterilization, or both, in accordance with a representative embodiment.

As will become clearer as the present description continues, a medical connector that is adapted to connect to a mating connector is blocked (or in a blocked state) when a component of the medical connector of the medical device is deformed so the medical connector cannot engage the mating connector. By contrast, a medical connector that is adapted to connect to a mating connector is unblocked (or in an unblocked state) when a component of the medical connector of the medical device is reformed so the medical connector can engage the mating connector. Various aspects of the method are described below in connection with various representative embodiments.

At 101, the state of the medical connector is assessed to determine whether or not the medical connector (sometimes referred to herein as a consumable) is blocked. If the medical connector is blocked, the method 100 proceeds to 102 to determine if the medical connector should be reprocessed. Specifically, when the consumable is blocked, and thus does not fit in the mating connector (e.g., monitor) at 101, the medical staff can either decide to reprocess the consumable at 103; or not to reprocess it. The method 100 then returns to 101.

At 103, the method continues with the cleaning, disinfecting, and if desired or required, sterilization of the medical connector. As noted above, the medical connector is disinfected in an automated washer and sterilized in most cases in an autoclave. These two steps are defined by the following standards: ISO 15883 for the automated cleaning; and ISO 17665 for the autoclave step. In accordance with various representative embodiments, the transformation temperature of the SMA element of the medical connector is selected to be greater than the maximum expected ambient room temperature (typically in the range of 25°C - 30°C), and no greater than the temperature required by ISO 15883, if only automated cleaning and disinfection is required; or no greater than the temperature required by ISO 17665 if steam sterilization in an autoclave is required.

Since the temperature of the washer and/or autoclave steps as required by ISO 15883 and ISO17665, respectively, are greater than the transformation temperature of the SMA in 103, the SMA is reformed to its original shape, and the method continues to 104 with the medical connector's returning to its unblocked state.

Once the medical connector is unblocked, the medical connector is clean, disinfected and if desired or required, sterilized, and can be used again by a clinician. As such, the method 100 continues to 105 where the medical connector is used to engage a mating connector.

At 106, after use, the medical connector is disconnected from its mating connector (e.g., at a monitor of a medical instrument). As alluded to above, and as described more fully below, disconnecting the medical connector deforms the SMA element, and at 107, the method 100 continues with the medical connector's being blocked again. Again, in the blocked state, the medical connector cannot be reconnected to its mating connector unless and until the SMA element is reformed to its original shape-where the transformation temperature required to reform the SMA element of the medical connector is selected to be no greater than the temperature required by ISO 15883, or ISO 17665 if sterilization in an autoclave is required. With the medical connector's being heated to at least these temperatures, the medical device is properly cleaned, disinfected and, if desired or required, sterilized. The medical connector is again in the unblocked state, and can be safely connected to it mating connector of the medical instrument.

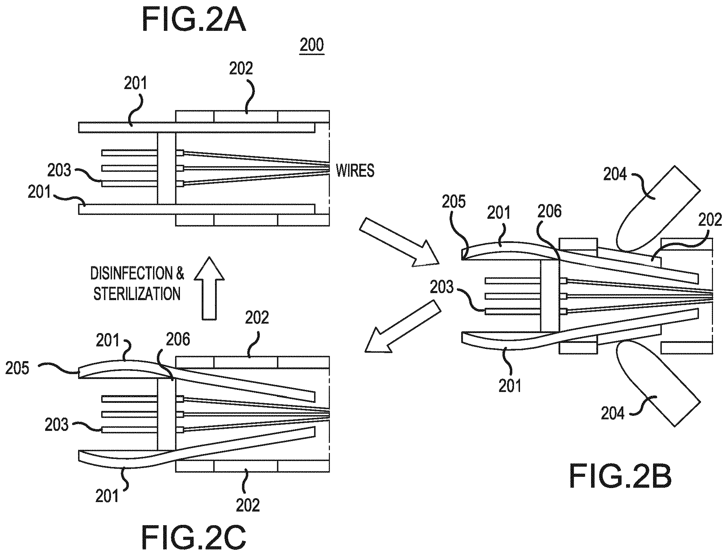

Figs. 2A-2C are simplified cross-sectional views of a medical connector 200 comprising an SMA element showing states of SMA element in three stages (A, B, C) of use of the according to a representative embodiment. Various aspects and details of the medical connector 200 are common to those described in the above portion of the Detailed Description. These aspects and details are not necessarily repeated, but nonetheless may be germane to the presently described representative embodiments.

As shown in Fig. 2A, the medical connector 200 comprises an SMA element 201, a button 202 and pins 203. As will become clearer as the present description continues, the medical connector 200 is in an unblocked state and can be connected to a mating connector (not shown) of a medical instrument for example. In the unblocked state, the SMA element 201 is in its original state and thus is not blocked from connecting to the mating connector. As noted above, and as described below, the medical connector 200 of Fig. 2A is cleaned, disinfected, and if desired or required, sterilized.

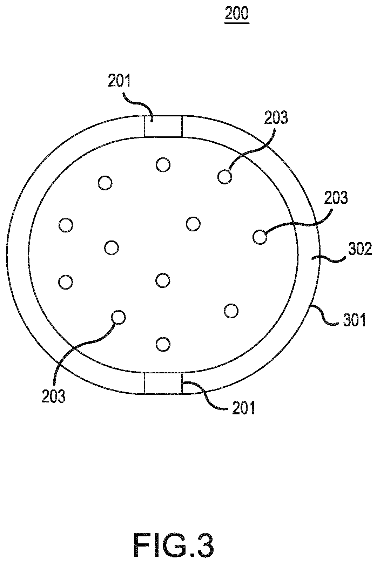

As shown in Fig. 2B, the button 202 is depressed, illustratively by finger/thumb 204. Notably, however, rather than finger/thumb 204, a tool or other similar device may be used to depress the button 202. As shown, the SMA element 201 is fixed in position at an extremity 205 of the medical connector 200 connector and approximately in a middle 206 of the SMA element 201. Stated somewhat differently, at the extremity 205 and approximately in the middle 206, the SMA element 201 is in direct contact with a non-moveable element (302 in Fig. 3) of the medical connector 200. The SMA element 201 is, therefore, fixed at these points.

Because of the depression of the button 202, the SMA element 201 is deformed with the portion near the extremity 205 and extending in a curved manner as shown. When the SMA element 201 is deformed, connection of the medical connector 200 to its mating connector (not shown) cannot be done. As such, the shape of the mating end of the medical connector 200 prevents the medical connector from mating with the mating connector (not shown) of the medical instrument or other mating connector (not shown). Accordingly, since the SMA element 201 is deformed, the medical connector 200 is in a blocked state, and cannot be reconnected to the medical instrument or other mating connector without having been cleaned disinfected, and, if desired or required, sterilized by at least the standards noted above.

As shown in Fig. 2C, the button 202 is released. However, because the SMA element 201 has been deformed, it stays in its deformed shape, and cannot be connected to its mating connector until it is heated to return to its original state. In accordance with a representative embodiment, the SMA element 201 may be one of a variety of shape memory alloys within the purview of the ordinarily skilled artisan. Just by way of illustration, and not limitation, the shape memory alloy used for the SMA element 201 may be one or more of Ag-Cd, Au-Cd, Cu-Al-Ni, Cu-Sn, Cu-Zn-(X), In-Ti, Ni-Al, Ni-Ti, Fe-Pt, Mn-Cu and Fe-Mn-Si alloys. These noted alloys are a group of metallic materials having the property of returning to a previously defined shape when subjected to an appropriate thermal procedure. The shape-memory effect occurs due to a temperature and stress dependent shift in the material's crystalline structure between two different phases, martensite (low temperature phase) and austenite (high temperature phase). The temperature, where the phase transformation occurs, is called the transformation temperature as noted above.

By the present teachings, the shape memory alloy used for the SMA element 201 is selected to ensure return to its original state (i.e., its shape in Fig. 2A) occurs only after being heated to a sufficient temperature to ensure cleaning, disinfecting and, if desired or required, sterilizing of the components of the medical connector. As such, the medical connector 200 in Fig. 2C undergoes a heating step at a sufficient temperature to disinfect, and if desired or required, sterilize the components of the medical connector 200 that engage the medical instrument or mating connector (not shown). After being sufficiently heated in the disinfection, and if desired or required, sterilization process, the SMA element 201 returns to its original shape as shown in Fig. 2A. In this, the unblocked state, the SMA element 201 does not obstruct the connection of the medical connector 200 to the medical equipment or mating connector (not shown), and can be reconnected to the mating connector in a cleaned, disinfected and if desired or required sterilized state for further use.

Accordingly, in connection with representative embodiments of Figs. 2A-2C, the medical connector 200 completes a cycle of disconnection and deformation of the SMA element 201; heating to clean, disinfect and, if desired or required, sterilize the components of the medical connection; and reformation of the SMA element 201 to its original state for further use by connecting to its mating connector or medical instrument (not shown). So, in its original state (Fig. 2A), the SMA element 101 is in *Shape 1* (unblocked). The medical connector 200 can therefore be inserted in its corresponding mating connector (e.g. a connector of a medical instrument such as a patient monitor). The SMA elements are fixed at two locations: at the extremity 205 of the medical connector 200, and approximately in the middle 206 of the SMA element 201, as shown in Fig. 2B. Buttons 202 are also added in the connector. The buttons 202 are located so a user manually presses (or causes to press using a tool) the buttons when removing the medical connector 200 from its medical instrument or mating connector. A locking mechanism, unlocked by the button, could potentially also be added such as the consumable cannot be removed unless the patient presses on the button. When the buttons 202 are pressed, an external force is applied to the SMA element 201. By this action, the SMA elements 201 are deformed in *Shape 2* (blocked). As shown in Fig. 2C, the SMA shape is such that the medical connector 200 cannot be reinserted back in its corresponding mating connector or medical instrument (not shown). Once disinfected, and if desired or required, sterilized (e.g., in a CSF), the SMA elements 201 are exposed to temperatures above the transformation temperature *Mₛ*. The SMA elements 201 therefore return to their original form, i.e. *Shape 1* (unblocked-Fig. 2A), and the medical connector can be reinserted back in its corresponding mating connector or medical instrument in a disinfected, or if desired or required, sterile state.

Fig. 3 is a simplified end-on view of the medical connector 300 device of Fig. 2A in an unblocked state, in accordance with a representative embodiment. Various aspects and details of the medical connector 300 are common to those described in the above portion of the Detailed Description. These aspects and details are not necessarily repeated, but may be germane to the presently described representative embodiments.

As shown in Fig. 3, the medical connector 200 has a comparatively circular cross-section. The medical connector 200 comprises an outer portion 301 that comprises SMA element 201 and a non-moveable element 302. In this unblocked state, the shape of the outer portion 301 of the medical connector is selected to mate with a corresponding outer portion of a mating connector (not shown) and to ensure easy mating of the pins 203 to their receiving counterparts of the mating connector or medical instrument. By contrast, the deformation of the SMA element 201 (e.g., by pressing buttons 202 shown in Fig. 2B) would result in the deformation of the outer portion 301 of the medical connector 200 (see Fig. 2B) in such a way that the outer portion 301 could not mate with the outer portion of the mating connector or medical instrument (not shown), and thereby, in its blocked state, the medical connector 200 could not mate with the mating connector or medical instrument. Again, to enable the medical connector 200 to mate with its mating connector, a heating step at a sufficient temperature to disinfect and, if desired or required, sterilize the components of the medical connector 200 that engage the medical instrument or device. After being sufficiently heated in the disinfection, and if desired or required, sterilization process, the SMA element 201 returns to its original shape as shown in Figs. 2A and 3. In this, the unblocked state, the SMA element 201 does not obstruct the connection of the medical connector 200 and the medical connector can again mate with the outer portion of the mating connector (not shown) in a disinfected or if desired or required sterilized state for further use.

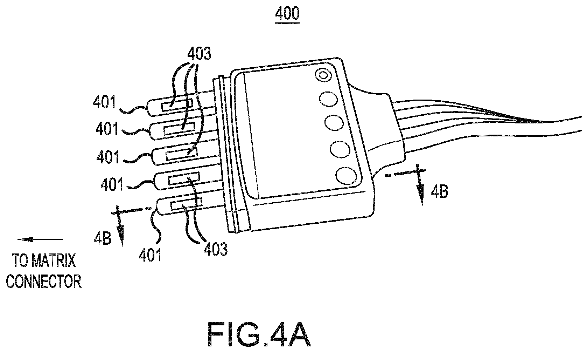

Fig. 4A is a perspective view of an electrocardiograph (ECG) lead set 400 (i.e., a medical connector) comprising electrically shielding electrodes in accordance with a representative embodiment. Various aspects and details of the lead set 400 are common to those described in the above portion of the Detailed Description. These aspects and details are not necessarily repeated, but may be germane to the presently described representative embodiments.

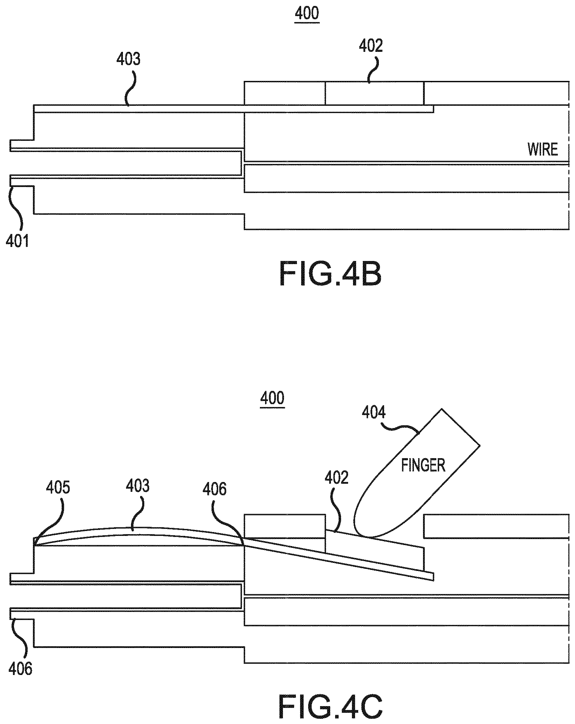

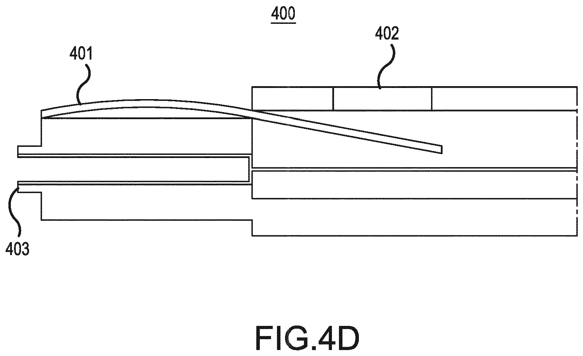

The lead set 400 includes a plurality of pins 401, and each of the plurality of pins 401 comprises an electrically shielding electrode 403. As described more fully below, in a representative embodiment, the electrically shielding electrodes 403 comprise a shape memory alloy that is in its original state (i.e., not deformed) in Fig. 4. With the electrically shielding electrodes 403 in their original state, the lead set 400 is unblocked and can be connected to a mating connector or medical instrument (not shown). As described more fully in connection with Figs. 4B-4D, when deformed during disconnection of the lead set 400 from a mating connector or medical instrument (not shown), the lead set 400 is blocked, and thereby preventing the lead set 400 from connecting to the mating connector of the ECG device until cleaning, disinfection, and, if desired or required, sterilization is completed. As such, and as described above, and below, the exposure to the heat in the cleaning, disinfection, and, if desired or required, sterilization sequence causes the electrically shielding electrodes 403 of SMA material to return to their original shape as in Fig. 4A (and as shown more clearly in Fig. 4B).

Figs. 4B-4D are simplified cross-sectional views of the ECG lead set 400 depicted in Fig. 4A showing states of SMA in three stages of use of the ECG lead set in accordance with a representative embodiment. Various aspects and details of the lead set 400 are common to those described in above portion of the Detailed Description. These aspects and details are not necessarily repeated, but may be germane to the presently described representative embodiments.

As shown in Fig. 4B, the lead set 400 comprises electrically shielding electrodes 403, a button 402 and pins 401. Notably, the electrically shielding electrode 403 is made of a shape memory alloy (SMA) such as described above. As will become clearer as the present description continues, in Fig. 4B the lead set 400 is in an unblocked state and can be connected to a mating connector or medical instrument. In the unblocked state, the electrically shielding electrode 403 is in its original state and thus is not blocked from connecting to the mating connector. As noted above, and as described below, the lead set 400 of Fig. 4B is cleaned, disinfected, and if desired or required, sterilized.

As shown in Fig. 4C, the button 402 is depressed, illustratively by a finger/thumb 404. Notably, however, rather than manual depression, a tool or other similar device may be used to depress the button 402. As shown, the electrically shielding electrode 403 is fixed in position at an extremity 405 of the lead set 400 and approximately in the middle 406 of the electrically shielding electrode 403. Again, and as noted above in the description of representative embodiments of Figs. 2A-3, at the extremity 405 of the lead set 400, the electrically shielding electrode 403 comprising the SMA material is in direct contact with non-moveable parts of the lead set 400. The electrically shielding electrode 403 is therefore fixed at these points. Because of the depression of the button 402, the electrically shielding electrode 403 is deformed with the portion near the extremity 405 extending in a curved manner as shown. When the electrically shielding electrode 403 is deformed, connection of the lead set 400 to its mating connector (not shown) cannot be done. As such, the shape of the mating end of the lead set 400 prevents its mating with the mating connector or medical instrument (not shown). Accordingly, since the SMA element of the electrically shielding electrode 403 is deformed, the lead set 400 is in a blocked state, and cannot be reconnected to the mating connector or medical instrument (not shown) without having been cleaned, disinfected, and if desired or required sterilized by at least the standards noted above.

As shown in Fig. 4D, the buttons 402 are released. However, because the electrically shielding electrode 403 has been deformed, it stays in its deformed shape, and cannot be connected to its mating connector until it is heated to return to its original state. In accordance with a representative embodiment, the electrically shielding electrode 403 may be made from one of a variety of shape memory alloys within the purview of the ordinarily skilled artisan. Just by way of illustration, and not limitation, the shape memory alloy used for the electrically shielding electrode 403 may be one or more of Ag-Cd, Au-Cd, Cu-Al-Ni, Cu-Sn, Cu-Zn-(X), In-Ti, Ni-Al, Ni-Ti, Fe-Pt, Mn-Cu and Fe-Mn-Si alloys. These noted alloys are a group of metallic materials having the property of returning to a previously defined shape when subjected to an appropriate thermal procedure. The shape-memory effect occurs due to a temperature and stress dependent shift in the material's crystalline structure between two different phases, martensite (low temperature phase) and austenite (high temperature phase). The temperature, where the phase transformation occurs, is called the transformation temperature noted above.

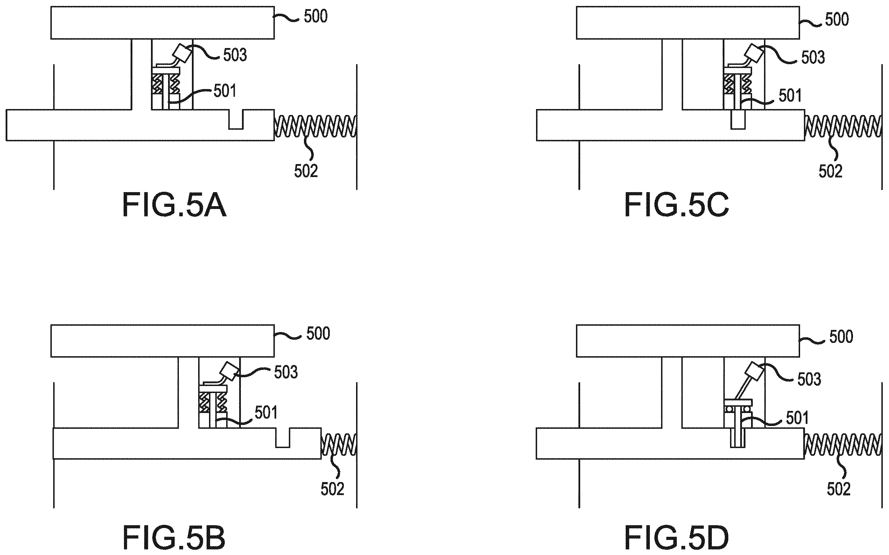

By the present teachings, the shape memory alloy used for the electrically shielding electrode 403 is selected to ensure return to its original state (i.e., its shape in Fig. 4B) occurs only after being heated to a sufficient temperature to ensure cleaning, disinfecting and, if desired or required, sterilizing of the components of the medical connector. As such, the lead set 400 in Fig. 4D undergoes a heating step at a sufficient temperature to disinfect and if desired or required sterilize the components of the lead set 400 that engage the medical instrument or device. After being sufficiently heated in the disinfection and if desired or required sterilization process, the electrically shielding electrode 403 comprising SMA material returns to its original shape as shown in Fig. 5A. In this, the unblocked state, the electrically shielding electrode 403 does not obstruct the connection of the lead set 400 to its mating connector or medical instrument (not shown), and can be reconnected to the mating connector or medical instrument in a disinfected or if desired or required sterilized state for further use.

Accordingly, in connection with representative embodiments of Figs. 4B-4D, the lead set 400 completes a cycle of disconnection and deformation of the electrically shielding electrode 403; heating to clean, disinfect, and if desired or required, sterilize the components of the medical connection; and reformation of the electrically shielding electrode 403 to its original state for further use by connecting to its mating connector (not shown) of the medical instrument. So, in its original state (Fig. 4B), the electrically shielding electrode 403 comprising the SMA material is in *Shape 1* (unblocked). The lead set 400 can therefore be inserted in its corresponding mating connector (e.g., a connector of a medical instrument such as a patient monitor). The electrically shielding electrode 403 comprising SMA elements are fixed at two locations: at the extremity 405 of the lead set 400, and approximately in the middle 406 of the electrically shielding electrode 403, as shown in Fig, 4C. Buttons 402 are also added in the connector. The buttons 402 are located so a user manually presses (or causes to press using a tool) the buttons when removing the lead set 400 from its mating connector (not shown). A locking mechanism, unlocked by the button 402, could potentially also be added such as the consumable cannot be removed unless the button 402 is pressed. When the buttons 402 are pressed, an external force is applied to the first spring loaded pin. By this action, the electrically shielding electrodes 403 comprising SMA material are deformed in *Shape 2* (blocked). As shown in Fig. 4D, the shape of the electrically shielding electrode 403 comprising the SMA material is such that the lead set 400 cannot be reinserted back in its corresponding mating connector of the medical instrument. Once disinfected, and if desired or required, sterilized (e.g., in a CSF), the of the electrically shielding electrode 403 comprising the SMA material are exposed to temperatures above the transformation temperature *Mₛ*. The shape of the electrically shielding electrode 403 comprising the SMA material therefore return to their original form, i.e. *Shape 1* (unblocked-Fig. 4B), and the medical connector can be reinserted back in its corresponding mating connector or medical instrument (not shown) in a disinfected or sterile state.

Figs. 5A-5H are simplified cross-sectional views of a medical connector 500 for a medical device showing states of SMA in various stages of use of the electrical connector in accordance with a representative embodiment. Various aspects and details of the medical connector 500 are common to those described in the above portion of the Detailed Description. These aspects and details are not necessarily repeated, but may be germane to the presently described representative embodiments.

In the previously described representative embodiments, an external force is applied by the user to deform the SMA element when the consumable is removed from its socket. The presently described representative embodiments describe an implementation where the consumable is automatically blocked when removed from its socket. Although the implementation of this embodiment requires additional elements (compared to the previous embodiments), no external force applied by the user is needed.

Figure 5A depicts a first spring loaded pin 501, which could be an actual electrical pin or an added pin that has no other functionality than to prevent the consumables from being inserted in a corresponding mating connector (not shown) when not reprocessed. Notably, the medical connector 500 in Fig. 5A is at an ambient temperature.

When the medical connector 500 is inserted in its corresponding mating connector, a second spring loaded pin 502 is pushed back in the medical connector 500, as shown in Figure 5B. Again, the medical connector 500 in Fig. 5B is at an ambient temperature. The first spring loaded pin 501 is also pushed back by the same action.

Once disconnected from a patient monitor or similar device, the second spring loaded pin 502 is again moved to the left, as depicted in Figure 5C. The first spring loaded pin 501 is then pushed down by a SMA or bimetal material element 503. The SMA or bimetal material element 503 is designed such that it pushes down on the first spring loaded pin 501 at ambient temperature. The first spring loaded pin 601 therefore also pushes down in the previous states described in Figs. 5A-5B. As shown in Fig. 6D, the first spring loaded pin 601 is pushed down by the SMA or bimetal material element 503 as shown in Fig. 6D. A second use is therefore impossible since the first spring loaded pin 601 prevents the electrical connector 600 from being properly inserted in its corresponding mating connector.

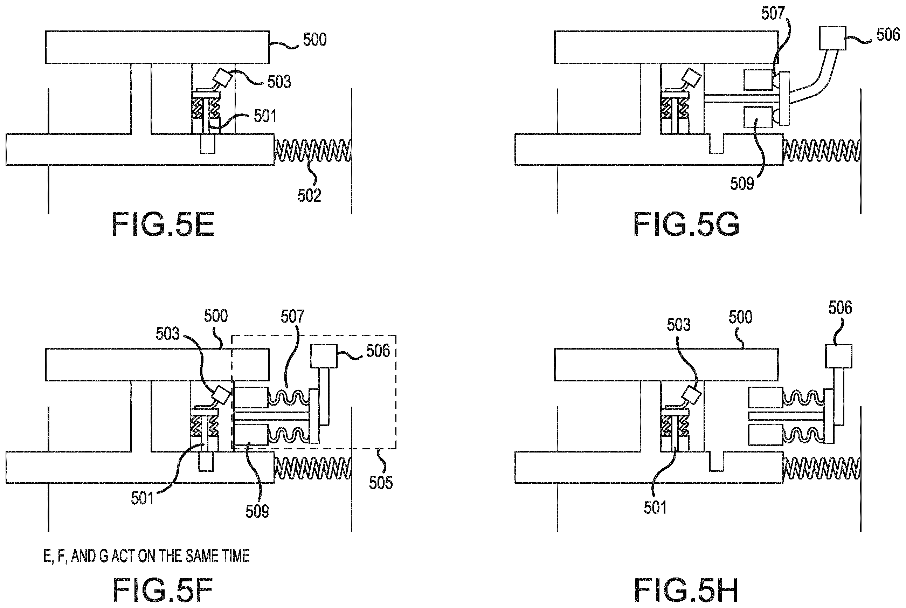

Turning to Fig. 5E, the medical connector 500 is disinfected in an automated washer and if desired or required sterilized in an autoclave, and as such is exposed to high temperatures, as explained in a section above. In this case, the SMA or bimetal material element 503 bends back because it is spring loaded, and the first spring loaded pin 501 is pushed back up, as shown in Figure 5E. At the same time, an additional spring loaded push mechanism 506 pushes the first spring loaded pin 501 to the left, as described in Figs. 5F and 5G. The spring in spring loaded push mechanism 506 comprises first spring 607 and second spring 609 as shown in Figs. 5F-5G comprise bimetal or SMA material that bends at high temperatures. When the medical connector returns to ambient temperature, the push mechanism 505 returns to its original state, as shown in Fig. 5H (which is identical to the state described in connection with Fig. 5A) and the cycle starts over again.

The illustrations of the representative embodiments described herein are intended to provide a general understanding of the structure of the various embodiments. Additionally, the illustrations are merely representational and may not be drawn to scale. Certain proportions within the illustrations may be exaggerated, while other proportions may be minimized.

## Claims

1. A method of ensuring a medical connector for a device is disinfected, or sterilized, the method comprising:
receiving the medical connector in a blocked state in which a component of the medical connector that comprises a shape-memory alloy, SMA; is deformed so that the medical connector cannot engage a mating connector; and
heating the medical connector to a sufficient temperature to change a shape of the SMA and thereby reform the component so that the medical connector can, in an unblocked state, engage the mating connector.

2. The method of claim 1, wherein the SMA is deformed to the blocked state upon removal of the medical connector from a mating connector.

3. The method of claim 1, wherein the SMA is disposed in a structure that surrounds pins (203,403,503) of the medical connector, and is deformed to the blocked state upon removal of the medical connector from a mating connector.

4. The method of claim 1, wherein the medical connector comprises electrically shielding electrodes comprising the SMA, wherein the electrically shielding electrodes are deformed to the blocked state upon removal of the medical connector from a mating connector.

5. The method of claim 4, wherein the heating of the SMA of the medical connector to the sufficient temperature to change the shape of the electrically shielding electrodes returns the medical connector to the unblocked state.

6. The method of claim 1, wherein the temperature is greater than or equal to 65°C.

7. The method of claim 1, wherein the temperature is in a range of 120°C -135°C.

8. The method of claim 1, wherein the SMA comprises one of Ag-Cd, Au-Cd, Cu-Al-Ni, Cu-Sn, Cu-Zn-(X), In-Ti, Ni-Al, Ni-Ti, Fe-Pt, Mn-Cu and Fe-Mn-Si alloys.

9. A medical device, comprising:
a medical connector (200, 400, 500) comprising a shape memory alloy, SMA, (201, 401, 501) adapted to engage a mating connector in an unblocked state, wherein upon disengaging the medical connector the SMA is in a blocked state so that the medical connector cannot engage the mating connector, the SMA being configured to change shape when the medical connector is heated to a sufficient temperature to enable the medical connector to engage the mating connector in the unblocked state.

10. The medical device of claim 9, wherein disengaging of the medical connector deforms the SMA to the blocked state.

11. The medical device of claim 9, wherein the medical connector comprises electrically shielding electrodes comprising the SMA, and the electrically shielding electrodes are deformed to the blocked state upon removal of the medical connector from a mating connector.

12. The medical device of claim 11, wherein the heating of the SMA of the medical connector to the sufficient temperature to change the shape of the electrically shielding electrodes returns the medical connector to the unblocked state.

13. The medical device of claim 9, wherein the temperature is greater than or equal to 65°C.

14. The medical device of claim 9, wherein the temperature is in a range of 120°C -135°C.

15. The medical device of claim 9, wherein the SMA comprises one of Ag-Cd, Au-Cd, Cu-Al-Ni, Cu-Sn, Cu-Zn-(X), In-Ti, Ni-Al, Ni-Ti, Fe-Pt, Mn-Cu and Fe-Mn-Si alloys.

## Patentansprüche

1. Verfahren zum Gewährleisten, dass ein medizinischer Verbinder für eine Vorrichtung desinfiziert oder sterilisiert wird, wobei das Verfahren umfasst:
Empfangen des medizinischen Verbinders in einem blockierten Zustand, in dem eine Komponente des medizinischen Verbinders, die eine Formgedächtnislegierung, SMA, umfasst, verformt ist, sodass der medizinische Verbinder nicht mit einem Gegenverbinder in Eingriff gelangen kann; und
Erhitzen des medizinischen Verbinders auf eine Temperatur, die ausreicht, um die Form der SMA zu verändern und dadurch die Komponente neu zu formen, sodass der medizinische Verbinder in einem nicht blockierten Zustand mit dem Gegenverbinder in Eingriff gelangen kann.

2. Verfahren nach Anspruch 1, wobei die SMA beim Entfernen des medizinischen Verbinders von einem Gegenverbinder in den blockierten Zustand verformt wird.

3. Verfahren nach Anspruch 1, wobei die SMA in einer Struktur angeordnet ist, die Stifte (203,403,503) des medizinischen Verbinders umgibt, und beim Entfernen des medizinischen Verbinders von einem Gegenverbinder in den blockierten Zustand verformt wird.

4. Verfahren nach Anspruch 1, wobei der medizinische Verbinder elektrisch abschirmende Elektroden umfasst, welche die SMA umfassen, wobei die elektrisch abschirmenden Elektroden beim Entfernen des medizinischen Verbinders von einem Gegenverbinder in den blockierten Zustand verformt werden.

5. Verfahren nach Anspruch 4, wobei das Erhitzen der SMA des medizinischen Verbinders auf eine Temperatur, die ausreicht, um die Form der elektrisch abschirmenden Elektroden zu ändern, den medizinischen Verbinder in den nicht blockierten Zustand zurückversetzt.

6. Verfahren nach Anspruch 1, wobei die Temperatur größer oder gleich 65°C ist.

7. Verfahren nach Anspruch 1, wobei die Temperatur in einem Bereich von 120°C - 135°C liegt.

8. Verfahren nach Anspruch 1, wobei die SMA eine von Ag-Cd, Au-Cd, Cu-Al-Ni, Cu-Sn, Cu-Zn-(X), In-Ti, Ni-Al, Ni-Ti, Fe-Pt, Mn-Cu und Fe-Mn-Si Legierungen umfasst.

9. Medizinische Vorrichtung, umfassend:
einen medizinischen Verbinder (200, 400, 500), der eine Formgedächtnislegierung, SMA, (201, 401, 501) umfasst, die angepasst ist, um in einem nicht blockierten Zustand mit einem Gegenverbinder in Eingriff zu gelangen, wobei sich die SMA beim Lösen des medizinischen Verbinders in einem blockierten Zustand befindet, sodass der medizinische Verbinder nicht mit dem Gegenverbinder in Eingriff gelangen kann, wobei die SMA konfiguriert ist, um ihre Form zu ändern, wenn der medizinische Verbinder auf eine Temperatur erhitzt wird, die ausreicht, um dem medizinischen Verbinder zu ermöglichen, in dem nicht blockierten Zustand mit dem Gegenverbinder in Eingriff zu gelangen.

10. Medizinische Vorrichtung nach Anspruch 9, wobei Lösen des medizinischen Verbinders die SMA in den blockierten Zustand verformt.

11. Medizinische Vorrichtung nach Anspruch 9, wobei der medizinische Verbinder elektrisch abschirmende Elektroden umfasst, welche die SMA umfassen, und die elektrisch abschirmenden Elektroden beim Entfernen des medizinischen Verbinders von einem Gegenverbinder in den blockierten Zustand verformt werden.

12. Medizinische Vorrichtung nach Anspruch 11, wobei das Erhitzen der SMA des medizinischen Verbinders auf eine Temperatur, die ausreicht, um die Form der elektrisch abschirmenden Elektroden zu ändern, den medizinischen Verbinder in den nicht blockierten Zustand zurückversetzt.

13. Medizinische Vorrichtung nach Anspruch 9, wobei die Temperatur größer oder gleich 65°C ist.

14. Medizinische Vorrichtung nach Anspruch 9, wobei die Temperatur in einem Bereich von 120°C -135°C liegt.

15. Medizinische Vorrichtung nach Anspruch 9, wobei die SMA eine von Ag-Cd, Au-Cd, Cu-Al-Ni, Cu-Sn, Cu-Zn-(X), In-Ti, Ni-Al, Ni-Ti, Fe-Pt, Mn-Cu und Fe-Mn-Si Legierungen umfasst.

## Revendications

1. Procédé permettant de garantir qu'un raccord médical pour un dispositif est désinfecté ou stérilisé, le procédé comprenant :
la réception du raccord médical dans un état bloqué dans lequel un composant du raccord médical qui comprend un alliage à mémoire de forme, SMA, est déformé de sorte que le raccord médical ne puisse pas venir en prise avec un raccord homologue ; et
le chauffage du raccord médical à une température suffisante pour changer une forme du SMA et ainsi reformer le composant de sorte que le raccord médical puisse, dans un état débloqué, venir en prise avec le raccord homologue.

2. Procédé selon la revendication 1, dans lequel le SMA est déformé en l'état bloqué lors du retrait du raccord médical d'un raccord homologue.

3. Procédé selon la revendication 1, dans lequel le SMA est disposé dans une structure qui entoure des broches (203, 403, 503) du raccord médical, et est déformé en l'état bloqué lors du retrait du raccord médical d'un raccord homologue.

4. Procédé selon la revendication 1, dans lequel le raccord médical comprend des électrodes de blindage électrique comprenant le SMA, dans lequel les électrodes de blindage électrique sont déformées en l'état bloqué lors du retrait du raccord médical d'un raccord homologue.

5. Procédé selon la revendication 4, dans lequel le chauffage du SMA du raccord médical à la température suffisante pour changer la forme des électrodes de blindage électrique ramène le raccord médical à l'état débloqué.

6. Procédé selon la revendication 1, dans lequel la température est supérieure ou égale à 65 °C.

7. Procédé selon la revendication 1, dans lequel la température est dans une plage de 120 °C-135 °C.

8. Procédé selon la revendication 1, dans lequel le SMA comprend l'un des alliages Ag-Cd, Au-Cd, Cu-Al-Ni, Cu-Sn, Cu-Zn-(X), In-Ti, Ni-Al, Ni-Ti, Fe-Pt, Mn-Cu et Fe-Mn-Si.

9. Dispositif médical, comprenant :
un raccord médical (200, 400, 500) comprenant un alliage à mémoire de forme, SMA, (201, 401, 501) conçu pour venir en prise avec un raccord homologue dans un état débloqué, dans lequel lors de la séparation du raccord médical, le SMA est dans un état bloqué de sorte que le raccord médical ne puisse pas venir en prise avec le raccord homologue, le SMA étant configuré pour changer de forme lorsque le raccord médical est chauffé à une température suffisante pour permettre au raccord médical de venir en prise avec le raccord homologue dans l'état débloqué.

10. Dispositif médical selon la revendication 9, dans lequel la séparation du raccord médical déforme le SMA en l'état bloqué.

11. Dispositif médical selon la revendication 9, dans lequel le raccord médical comprend des électrodes de blindage électrique comprenant le SMA, et les électrodes de blindage électrique sont déformées en l'état bloqué lors du retrait du raccord médical d'un raccord homologue.

12. Dispositif médical selon la revendication 11, dans lequel le chauffage du SMA du raccord médical à la température suffisante pour changer la forme des électrodes de blindage électrique ramène le raccord médical à l'état débloqué.

13. Dispositif médical selon la revendication 9, dans lequel la température est supérieure ou égale à 65 °C.

14. Dispositif médical selon la revendication 9, dans lequel la température est dans une plage de 120 °C-135 °C.

15. Dispositif médical selon la revendication 9, dans lequel le SMA comprend l'un des alliages Ag-Cd, Au-Cd, Cu-Al-Ni, Cu-Sn, Cu-Zn-(X), In-Ti, Ni-Al, Ni-Ti, Fe-Pt, Mn-Cu et Fe-Mn-Si.
